# EUROPEAN PATENT SPECIFICATION

(11) **EP 2 714 086 B1**
(45) Date of publication and mention of the grant of the patent: **10.12.2014**
(21) Application number: 12715969.7
(22) Date of filing: 17.04.2012
(51) Int. Cl.: A61P 9/10, A61K 31/765, A61K 45/06, A61K 31/12, A61K 31/353

(54) **POLYPHENOL COMPOSITION FOR PREVENTING OR TREATING ATHEROSCLEROSIS**
POLYPHENOL-ZUSAMMENSETZUNG ZUR VORBEUGUNG ODER BEHANDLUNG VON ATHEROSKLEROSE
COMPOSITION POLYPHÉNOLIQUE POUR LA PRÉVENTION OR LE TRAITEMENT DE L'ATHÉROSCLÉROSE

(30) Priority: 27.05.2011 EP 11167805
(43) Date of publication of application: 09.04.2014
(73) Proprietor: Unilever PLC, London, Greater London EC4Y 0DY (GB); Unilever N.V., 3013 AL Rotterdam (NL)
(72) Inventor: FOWLER, Mark, Ian, Bedford Bedfordshire MK44 1LQ (GB); JENKINS, Gail, Bedford Bedfordshire MK44 1LQ (GB); WAINWRIGHT, Linda, Jane, Bedford Bedfordshire MK44 1LQ (GB)
(74) Representative: Acham, Nicholas Clive
(86) International application number: PCT/EP2012/056980
(87) International publication number: WO 2012/163589

(56) References cited:
- EP-A1- 1 852 430
- EP-A2- 0 713 706
- WO-A1-97/09877
- US-A1- 2006 135 446
- ERNST E ET AL: "BLOOD RHEOLOGY IN PATIENTS WITH TRANSIENT ISCHEMIC ATTACKS", STROKE, LIPPINCOTT WILLIAMS & WILKINS, US, vol. 19, no. 5, 1 May 1988 (1988-05-01), pages 634-636, XP008032166, ISSN: 0039-2499
- "Beneficial effect of a novel grape seed proanthocyanidin extract in atherosclerosis models", FREE RADICAL BIOLOGY AND MEDICINE, ELSEVIER SCIENCE, US, vol. 27, 1 January 1999 (1999-01-01), page S45, XP027348351, ISSN: 0891-5849 [retrieved on 1999-01-01]
- YAMAKOSHI J; KATAOKA S; KOGA T; ARIGA T: "Proanthocyanidin-rich extract from grape seeds attenuates the development of aortic atherosclerosis in cholesterol-fed rabbits", ATHEROSCLEROSIS, vol. 142, no. 1, January 1999 (1999-01), pages 139-149, XP002654052, ISSN: 0021-9150
- QIN L ET AL: "Effects and underlying mechanisms of curcumin on the proliferation of vascular smooth muscle cells induced by Chol:MbetaCD", BIOCHEMICAL AND BIOPHYSICAL RESEARCH COMMUNICATIONS, ACADEMIC PRESS INC. ORLANDO, FL, US, vol. 379, no. 2, 6 February 2009 (2009-02-06), pages 277-282, XP027552655, ISSN: 0006-291X, DOI: DOI:10.1016/J.BBRC.2008.12.038 [retrieved on 2009-02-06]
- JUNETSU OGASAWARA ET AL: "Oligonol, an oligomerized lychee fruit-derived polyphenol, activates the Ras/Raf-1/MEK1/2 cascade independent of the IL-6 signaling pathway in rat primary adipocytes", BIOCHEMICAL AND BIOPHYSICAL RESEARCH COMMUNICATIONS, vol. 402, no. 3, 1 November 2010 (2010-11-01), pages 554-559, XP55021899, ISSN: 0006-291X, DOI: 10.1016/j.bbrc.2010.10.082
- OGASAWARA JUNETSU ET AL: "Oligonol, A New Lychee Fruit-derived Low-molecular Form of Polyphenol, Enhances Lipolysis in Primary Rat Adipocytes through Activation of the ERK1/2 pathway", PHYTOTHERAPY RESEARCH, vol. 23, no. 11, November 2009 (2009-11), pages 1626-1633, XP002680235, ISSN: 0951-418X, DOI: DOI:10.1002/PTR.2846

## Description

This invention relates to the provision of an oral composition for preventing or treating atherosclerosis.

Cardiovascular disease (a class of diseases that involve the heart or blood vessels) contributes to over a third of global deaths and is currently the leading cause of death in developing countries. The risk of heart disease increases as you age and this term technically refers to any disease that affects the cardiovascular system. It is usually used to refer to those related to atherosclerosis (arterial disease). Berliner et al. (Circulation, 91, 2488-2496 (1995)) disclose that the clinical events resulting from atherosclerosis are directly related to the oxidation of lipids in low density lipoproteins (LDLs) that become trapped in the extracellular matrix of the sub-endothelial space.

Gems et al (Mechanisms of Ageing and Development, 126, 381-387 (2005)) describes the green theory of ageing in which it is suggested that accumulation of lipophilic toxic by-products from stochastic errors of metabolism which are therefore not recognisable to the cell lead to molecular damage and ageing. Gems et al further describe how the smooth endoplasmic reticulum, a eukaryotic organelle, acts as a cellular filter deploying phase 1 and phase 2 metabolism to mobilise and excrete the lipophilic toxins. Phase 1 metabolism results in addition of chemically reactive functional groups which allow further metabolism of otherwise unreactive lipophilic compounds. Phase 2 metabolism involves the addition of side groups which increase solubility aiding excretion. Cytochrome P450 and short-chain dehydrogenases or reductases are agents of phase 1 metabolism in mammalian cells. Uridine 5'-diphospho-glucuronosyltransferases (also known as UDP-glucuronosyltransferases or UGT's), which are glycosyltransferases that catalyze addition of the glycosyl group from a uridine-5'-triphosphate (UTP)-sugar to a small hydrophobic molecule (known as the glucuronidation reaction), are important agents of phase 2 metabolism.

Mattson et al (Dose-Response, 5, 174-186 (2007) disclose that curcumin is a potent inducer of numerous nuclear factor erythroid-derived 2 (Nrf2) transcription factor dependent phase 2 enzymes involved in xenobiotic detoxification. Lee et al (Journal of Biochemistry and Molecular Biology, 37, 2, 139-143 (2004)) describe enzymes induced by curcumin include the antioxidant response element (ARE) targets: NQ01,4 γ-glutamylcysteine synthetase (GGCS), Heme Oxygenase-1 (HO-1), glutathione transferases (GST), glucuronosyltransferases, and epoxide hydrolases.

Murashima et al (Biofactors, 22, 1-4, 271-5 (2004)) describe that other Nrf2 mediated effects include inhibition of low density lipoprotein (LDL) oxidation. Marco et al (N. Engl. J. Med., 337, 408-416 (1997)) describe how atherosclerosis develops from LDL becoming oxidized by free radicals, particularly reactive oxygen species. Therefore activation of Nrf2 therefore should lead to inhibition of oxidation of LDL with Heme Oxygenase-1 (HO-1) (a phase II detox enzyme) an indicator or end marker of Nrf2 activation.

EP 1 852 430 A discloses a composition containing as its main component proanthocyanidin oligomer to which a substance having a phloroglucinol ring structure or resorcinol ring structure has been bonded, which is obtainable by heating, for example at 90 to 100 degrees centigrade for 1 to 4 hours, plant materials comprising proanthocyanidin polymer or aqueous extract thereof comprising proanthocyanidin polymer, with a substance having a phloroglucinol ring structure or resorcinol ring structure in an acidic aqueous solution. The resulting mixture may then be filtered, concentrated, fractionated or dried using standard techniques known to the skilled person. A best mode for preparing the composition is disclosed in paragraphs [0015] to [0023] of EP 1 852 430 A and is incorporated herein by reference. Such a composition has been reduced in molecular weight compared to the proanthocyanidin polymer feed stock to such a level that the oligomer can be more easily absorbed into a living body. The composition comprises compounds of structure (I): 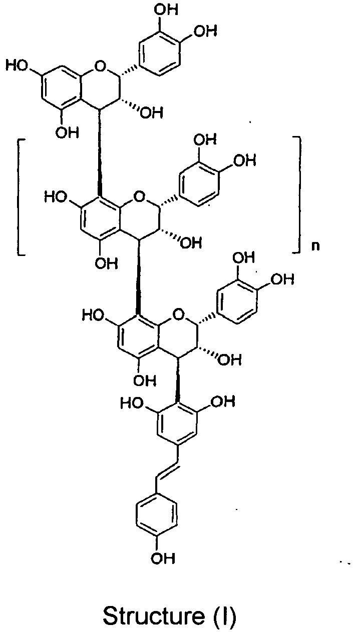 wherein n is 0 or an integer of 1 or 2, and/or structure (II): 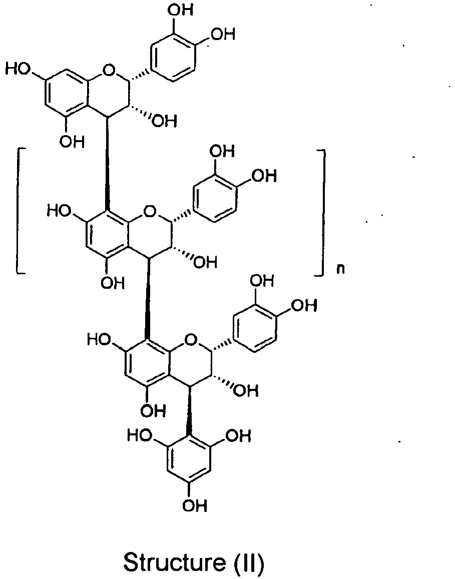 wherein n is 0 or an integer of 1 or 2. Oligomers of structure (I) and (II) are obtained by fractionating the aforementioned composition of EP 1 852 430 A using standard fractionation methods known to the skilled person in the art.

The plant material comprising proanthocyanidin polymer or extract thereof comprising proanthocyanidin polymer may be selected from the group consisting of grape, pine, Chamaecyparis obtuse, camphor tree, wax myrtle, cacao, date plum, banana, Chinese quince, apple, hawthorn, Lychi chinensis, Myrica rubra and Cinnamomi cortex. The substance having a phloroglucinol ring structure or resorcinol ring structure may be selected from the group consisting of resveratrol, phloroglucinol, flavonoid and flavanoid. The substance having a phloroglucinol ring structure or resorcinol ring structure may also be selected from the group consisting of green tea, fresh tea leaves, grape seed, grape seed coat, cube gambir, red algae and extracts thereof.

EP 1 852 430 A further discloses that such a composition exhibits significant antioxidative activity in a 1,1-diphenyl-2-picrylhydrazyl (DPPH) radical-scavenging activity assay and a Trolox Equivalent Antioxidant Capacity (TEAC) assay. It is also disclosed that application of such compositions leads to improved cell viability under ultra-violet radiation, significant anti-oxidant activity to blood serum and liver lipid peroxides (in mice) and reductions in blood serum levels of the liver enzymes glutamyl oxaloacetic transaminase (GOT) and glutamyl pyruvic transaminase (GPT). GOT and GPT are involved in transferring the amino group of amino acids from alpha-amino acids to alpha-keto acids. Transaminases are stored in the liver and are normally present only at low levels in blood serum. They are released into blood when the liver cells are damaged, thus detection of high levels of transaminases in blood serum is evidence of liver cell damage.

A composition according to EP 1 852 430 A is available commercially under the name Oligonol™ (Cognis) and comprises proanthocyanidin oligomer to which a substance having a phloroglucinol ring structure or resorcinol ring structure has been bonded, which is obtainable by heating plant materials comprising proanthocyanidin polymer or extract thereof comprising proanthocyanidin polymer, with a substance having a phloroglucinol ring structure or resorcinol ring structure in an acidic aqueous solution., the plant material being an extract produced from a combination of fruit from Lychi chinensis and green leaf of Camellia sinensis.

The inventors have observed that a combination of a composition according to EP 1 852 430 A and curcumin synergistically boosts the performance of phase 2 detoxification enzymes.

### Summary of the invention

Thus in a first aspect of the invention, an oral composition for preventing or treating atherosclerosis according to claim 1 is provided.

The composition may comprise 10-5000, preferably 50-2500, most preferably 100-300 mg curcumin and 10-5000, preferably 50-2500, most preferably 100-300 mg composition containing as its main component proanthocyanidin oligomer to which a substance having a phloroglucinol ring structure or resorcinol ring structure has been bonded, which is obtainable by heating plant materials comprising proanthocyanidin polymer or aqueous extract thereof comprising proanthocyanidin polymer, with a substance having a phloroglucinol ring structure or resorcinol ring structure in an acidic aqueous solution.

The weight ratio of curcumin to composition containing as its main component proanthocyanidin oligomer to which a substance having a phloroglucinol ring structure or resorcinol ring structure has been bonded, which is obtainable by heating plant materials comprising proanthocyanidin polymer or aqueous extract thereof comprising proanthocyanidin polymer, with a substance having a phloroglucinol ring structure or resorcinol ring structure in an acidic aqueous solution may be 50:1 to 1:50, preferably 20:1 to 1:20, most preferably 15:1 to 1:15.

In a second aspect of the invention, a composition according the first aspect of the invention is provided for use as a medicament.

In a second aspect of the invention, a composition according to the first aspect of the invention is provided for use in preventing or treating atherosclerosis.

### Summary of the figures

The invention is illustrated with reference to:

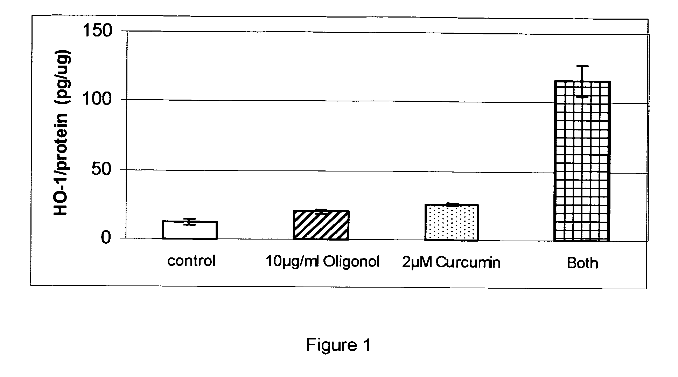

Figure 1 shows heme oxygenase 1 (HO-1) (pg per µg protein) for control (untreated human umbilical vein endothelial cells), 2 µM curcumin, 10 µg/ml Oligonol™, and a combination of 2 µM curcumin and 10 µg/ml Oligonol™.

### Detailed description of the invention

Oral compositions of the invention may be in the form of capsules, pills, tablets, granules, solutions, suspensions or emulsions. The amount of curcumin or composition containing as its main component proanthocyanidin oligomer to which a substance having a phloroglucinol ring structure or resorcinol ring structure has been bonded, which is obtainable by heating plant materials comprising proanthocyanidin polymer or extract thereof comprising proanthocyanidin polymer, with a substance having a phloroglucinol ring structure or resorcinol ring structure in an acidic aqueous solution obtainable by heating plant material being an extract produced from a combination of fruit from Lychi chinensis and green leaf of Camellia sinensis in an acidic aqueous solution is Oligonol™ in a capsule, pill or tablet will not be in excess of the recommended daily limit for an adult person. Preparation of compositions of the invention in the aforementioned oral formats is well known to the person skilled in the art.

### Example 1

Huvec cells, also known as human umbilical vein endothelial cells, (TCS Biologicals) were cultured and passaged in endothelial growth medium (EGM-2) (Biowhittaker) supplemented with heparin, vascular endothelial growth factor (VEGF), gentamicin sulphate, ascorbic acid, human endothelial growth factor (HEGF), hydrocortisone, (human fibroblast growth factor B (HFGF-B), long R insulin-like growth factor 1 (R3-IGF-1) and foetal bovine serum (FBS). Cells were routinely plated out in 6-well tissue culture dishes, at a seeding density of ∼5000 cells/cm² in 2 ml complete medium per well 24 hours before starting the experiment and incubated at 37°C in 5% CO₂.

The vascular endothelium, which lines the inner side of blood vessels, is one of the largest secretory tissues of the body. Huvec cells, also known as human umbilical vein endothelial cells, are used as an in-vitro model, as described in Bachettia et al. (Pharmacological Research, 42, 1 (2000)), to examine the potential of different ingredients to help in the prevention and/or therapy of cardiovascular diseases.

The cells were treated with 5 µM 4-hydroxynonenal and aqueous solutions of the test substances for 24 hours and then the level of heme oxygenase 1 measured.

The cells were harvested by adding 1 ml of trypsin/EDTA solution (Invitrogen) and incubated at 37°C until detached. The cells were then centrifuged immediately at 13000 rpm for 10 minutes and the supernatant discarded. The resulting cell pellet was washed with 500 µl of Dulbecco's phosphated buffer solution (PBS) and centrifuged as before. The supernatant was discarded as before and the cell pellet stored at -20°C prior to cell lysis.

All cell pellets were lysed on ice for 30 minutes in 1 ml cell lysis buffer per 2.5 x 10⁶ cells. The lysis buffer contained 1% nonyl phenoxypolyethoxylethanol (Tergitol-type NP-40), 0.1% sodium deoxycholate, 0.1% sodium dodecyl sulphate (SDS), 6 mM sodium chloride and 0.05M tris(hydroxymethyl)aminomethane at pH 7.6. Protease inhibitor cocktail (1000X; Sigma P8340) was added prior to use at a level of 10 µl/ml of lysis buffer. The partially lysed cell pellets were completely homogenised with a pellet pestle and unwanted cell debris removed by centrifugation for 20 minutes at 20,000g at 4°C. The clarified cell lysate was frozen at -80°C until needed.

Cell lysate was examined for cytotoxicity using the Promega CytoTox 96 nonradioactive cytotoxicity assay. This assay quantitatively measures lactate dehydrogenase (LDH) released upon cell lysis and is a good indication of cell viability. 50 µl of cell lysate or control medium was added to duplicate wells of a 96 well plate. 50 µl of CytoTox reagent was added and mixed well. The plate was incubated in the dark at room temperature for 30 minutes after which 50 µl of stop solution was added to each well and the absorbance of the plate read at 492 nm. Any test samples giving an absorbance value of more than double that of the control medium was considered to be cytotoxic. No results have been included from samples that showed any signs of cytotoxicity.

The total protein concentration of each cell lysate was measured using the Pierce BCA protein assay kit so that the response to effect of the test substances could be normalised to ng protein. A set of eight standard solutions ranging from 0 to 1200 µg/ml protein was prepared from the supplied 2 mg/ml bovine serum albumin (BSA) stock solution. 10 µl of standard or cell lysate was added to duplicate wells of a flat-bottomed 96-well plate. The reagent solution was prepared according to the kit instructions from 50 parts reagent A and 1 part reagent B. 200 µl of the final reagent was added to each well. The plate was mixed, covered and incubated at 37°C for 30 minutes and absorbance read at 562 nm. A protein standard curve was constructed and used to determine the protein concentration of each cell lysate.

Huvec cell lysate was diluted in sample diluent, and 100 µl transferred to a precoated anti-human heme oxygenase 1 immunoassay plate (Assay Designs). A 7-point recombinant heme oxygenase 1 standard curve, ranging from 25 to 0.39 ng/ml, was also prepared in sample diluent. The immunoassay plate was incubated at room temperature for 30 minutes, washed six times with wash buffer, and incubated for a further 60 minutes at room temperature with 100 µl/well of anti-heme oxygenase 1 antibody solution. The plate was washed as above and incubated for 30 minutes at room temperature with 100 µl/well of horseradish peroxidise conjugate solution. Again the plate was washed as above and 100 µl/well of tetramethylbenzidine substrate added to each well and stored for 15 minutes at room temperature in the dark. Following the addition of 100 µl/well of stop solution, the absorbance was read at 450 nm and the unknown biopsy levels of heme oxygenase 1 were extrapolated from the standard curve.

Figure 1 shows heme oxygenase 1 (HO-1) (pg per µg protein) for a control (untreated Huvec's), 2 µM curcumin, 10 µg/ml Oligonol™, and a combination of 2 µM curcumin and 10 µg/ml Oligonol™. Both curcumin and Oligonol™ appear to increase the level of heme oxygenase 1 and thus boost phase 2 metabolism. However a combination of 10 µM curcumin and 10 µg/ml Oligonol™ appears to significantly and synergistically increase heme oxygenase 1 levels and hence will reduce LDL oxidation indirectly reducing the onset of atherosclerosis.

It may be activated using a number of different routes (via induction of Nrf2 gene expression and nuclear translocation). Nrf2 transcription factors up-regulates the messenger ribonucleic acid (mRNA), protein and activity for HO-1 and it is believed that both Oligonol™ and curcumin target and activate different parts of the Nrf2 pathway. The results now show that this up-regulation occurs in a synergistic manner, for example, through conformational changes or increases in phosphorylation which may result in increases in nuclear translocation of Nrf2 transcription factors.

## Claims

1. An oral composition for preventing or treating atherosclerosis, the composition comprising curcumin and a composition containing as its main component proanthocyanidin oligomer to which a substance having a phloroglucinol ring structure or resorcinol ring structure has been bonded, which is obtainable by heating plant materials comprising proanthocyanidin polymer or aqueous extract thereof comprising proanthocyanidin polymer, with a substance having a phloroglucinol ring structure or resorcinol ring structure in an acidic aqueous solution, obtainable by heating plant material being an extract produced from a combination of fruit from Lychi chinensis and green leaf of Camellia sinensis in an acidic aqueous solution is Oligonol™.

2. A composition according to claim 1 comprising 10-5000, preferably 50-2500, most preferably 100-300 mg curcumin and 10-5000, preferably 50-2500, most preferably 100-300 mg composition containing as its main component proanthocyanidin oligomer to which a substance having a phloroglucinol ring structure or resorcinol ring structure has been bonded, which is obtainable by heating plant materials comprising proanthocyanidin polymer or aqueous extract thereof comprising proanthocyanidin polymer, with a substance having a phloroglucinol ring structure or resorcinol ring structure in an acidic aqueous solution.

3. A composition according to claim 1 or claim 2 wherein the weight ratio of curcumin to composition containing as its main component proanthocyanidin oligomer to which a substance having a phloroglucinol ring structure or resorcinol ring structure has been bonded, which is obtainable by heating plant materials comprising proanthocyanidin polymer or aqueous extract thereof comprising proanthocyanidin polymer, with a substance having a phloroglucinol ring structure or resorcinol ring structure in an acidic aqueous solution 50:1 to 1:50, preferably 20:1 to 1:20, most preferably 15:1 to 1:15.

4. A composition according to any one of claims 1 to 3 for use as a medicament.

5. A composition according to any one of claims 1 to 3 for use in preventing or treating atherosclerosis.

## Patentansprüche

1. Orale Zubereitung für die Verhinderung oder Behandlung von Atherosklerose, wobei die Zubereitung Folgendes aufweist: Curcumin und eine Zusammensetzung, die als deren Hauptbestandteil ein Proanthocyanidin-Oligomer enthält, an das eine Substanz mit einer Phloroglucinol-Ringstruktur oder Resorcinol-Ringstruktur gebunden ist,
die durch Erhitzen von Pflanzenmaterialien, die Proanthocyanidin-Polymer aufweisen, oder eines wässrigen Extraktes davon, der Proanthocyanidin-Polymer aufweist, mit einer Substanz mit einer Phloroglucinol-Ringstruktur oder Resorcinol-Ringstruktur in einer sauren wässrigen Lösung erhalten werden kann, die durch Erhitzen von Pflanzenmaterial erhalten werden kann, das ein Extrakt ist, der aus einer Kombination von Früchten von Lychi chinensis und grünen Blättern von Camellia sinsensis in einer sauren wässrigen Lösung erzeugt wurde, der Oligonol^{®} ist.

2. Zubereitung nach Anspruch 1,
die 10 bis 5000, vorzugsweise 50 bis 2500, besonders bevorzugt 100 bis 300 mg Curcumin und 10 bis 5000, vorzugsweise 50 bis 2500, besonders bevorzugt 100 bis 300 mg Zusammensetzung enthält, die als deren Hauptbestandteil Proanthocyanidin-Oligomer enthält, an das eine Substanz mit einer Phloroglucinol-Ringstruktur oder Resorcinol-Ringstruktur gebunden ist,
die durch Erhitzen von Pflanzenmaterialien, die Proanthocyanidin-Polymer aufweisen, oder eines wässrigen Extraktes davon, der Proanthocyanidin-Polymer aufweist, mit einer Substanz mit einer Phloroglucinol-Ringstruktur oder Resorcinol-Ringstruktur in einer sauren wässrigen Lösung erhalten werden kann.

3. Zubereitung nach Anspruch 1 oder 2,
wobei das Gewichtsverhältnis zwischen Curcumin und der Zusammensetzung, die als deren Hauptbestanteil Proanthocyanidin-Oligomer enthält, an das eine Substanz mit einer Phloroglucinol-Ringstruktur oder Resorcinol-Ringstruktur gebunden ist, die durch Erhitzen von Pflanzenmaterialien, die Proanthocyanidin-Polymer aufweisen, oder eines wässrigen Extraktes davon, der Proanthocyanidin-Polymer aufweist, mit einer Substanz mit einer Phloroglucinol-Ringstruktur oder Resorcinol-Ringstruktur in einer sauren wässrigen Lösung erhalten werden kann, 50:1 bis 1:50, vorzugsweise 20:1 bis 1:20, besonders bevorzugt 15:1 bis 1:15 beträgt.

4. Zusammensetzung nach einem der Ansprüche 1 bis 3 für die Verwendung als Medikament.

5. Zusammensetzung nach einem der Ansprüche 1 bis 3 für die Verwendung bei der Verhinderung oder Behandlung von Atherosklerose.

## Revendications

1. Composition orale pour prévenir ou traiter l'athérosclérose, la composition comprenant de la curcumine et une composition contenant comme composant principal un oligomère de proanthocyanidine auquel une substance ayant une structure cyclique de phloroglucinol ou une structure cyclique de résorcinol a été liée, qui peut être obtenue par chauffage de matières végétales comprenant un polymère de proanthocyanidine ou d'un extrait aqueux de celles-ci comprenant un polymère de proanthocyanidine, avec une substance ayant une structure cyclique de phloroglucinol ou une structure cyclique de résorcinol dans une solution aqueuse acide, pouvant être obtenue par chauffage d'une matière végétale qui est un extrait produit à partir d'une combinaison de fruit de Lychi chinensis et de feuille verte de Camellia sinensis dans une solution aqueuse acide est Oligonol™.

2. Composition selon la revendication 1 comprenant 10-5000, de préférence 50-2500, de manière particulièrement préférable 100-300 mg de curcumine et 10-5000, de préférence 50-2500, de manière particulièrement préférable 100-300 mg de composition contenant comme composant principal un oligomère de proanthocyanidine auquel une substance ayant une structure cyclique de phloroglucinol ou une structure cyclique de résorcinol a été liée, qui peut être obtenue par chauffage de matières végétales comprenant un polymère de proanthocyanidine ou d'un extrait aqueux de celles-ci comprenant un polymère de proanthocyanidine, avec une substance ayant une structure cyclique de phloroglucinol ou une structure cyclique de résorcinol dans une solution aqueuse acide.

3. Composition selon la revendication 1 ou la revendication 2 où le rapport en poids de la curcumine à la composition contenant comme composant principal un oligomère de proanthocyanidine auquel une substance ayant une structure cyclique de phloroglucinol ou une structure cyclique de résorcinol a été liée, qui peut être obtenue par chauffage de matières végétales comprenant un polymère de proanthocyanidine ou d'un extrait aqueux de celles-ci comprenant un polymère de proanthocyanidine, avec une substance ayant une structure cyclique de phloroglucinol ou une structure cyclique de résorcinol dans une solution aqueuse acide 50:1 à 1:50, de préférence 20:1 à 1:20, de manière particulièrement préférable 15:1 à 1:15.

4. Composition selon l'une quelconque des revendications 1 à 3 destinée à être utilisée comme médicament.

5. Composition selon l'une quelconque des revendications 1 à 3 destinée à être utilisée dans la prévention ou le traitement de l'athérosclérose.
